(19) Europäisches Patentamt
European Patent Office
Office européen des brevets

(11) Publication number: **0 071 279**
**B1**

# (12) EUROPEAN PATENT SPECIFICATION

(45) Date of publication of patent specification: **03.09.86**

(21) Application number: **82106960.6**

(22) Date of filing: **30.07.82**

(51) Int. Cl.$^4$: **C 07 C 127/22,**
C 07 D 213/81,
C 07 D 213/82,
C 07 D 233/96, A 01 N 47/34

## (54) Substituted N-aroyl N'-phenyl urea compounds.

(30) Priority: **30.07.81 US 288620**

(43) Date of publication of application:
**09.02.83 Bulletin 83/06**

(45) Publication of the grant of the patent:
**03.09.86 Bulletin 86/36**

(84) Designated Contracting States:
**BE DE FR GB IT NL SE**

(56) References cited:
**EP-A-0 003 099**
**EP-A-0 008 435**
**EP-A-0 023 884**
**DE-A-2 726 684**
**DE-A-2 848 794**
**US-A-4 110 469**

(73) Proprietor: **THE DOW CHEMICAL COMPANY**
**2030 Dow Center Abbott Road P.O. Box 1967**
**Midland, MI 48640 (US)**

(72) Inventor: **Rigterink, Raymond Henry**
**303 West Meadowbrook**
**Midland Michigan 48640 (US)**
Inventor: **Sbragia, Ronald Joseph**
**20 Malibu Court**
**Clayton California 94517 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 279 B1

**Description**

This invention relates to novel substituted N-aroyl N'-phenyl ureas, a process for producing them, insecticidal compositions containing them and a method for controlling certain insects.

Various insecticidal derivatives of urea are known in the art, such as, for example, U.S. Patents 4,173,638; 4,005,223; 4,170,657; 4,139,636; 4,089,975 and German Patent Application 3,003,113.

From an article by Wellinga et al. entitled "Synthesis and Laboratory Evaluation of 1-(2,6-Disubstituted benzoyl)-3-phenylureas, a New Class of Insecticides", Agricultural and Food Chemistry *21* (3), 348—354 (1973) phenyl ureas are known containing one or two substituents in the aniline ring. In this reference it is stated that compounds with a tri-substitution in the aniline ring have a poor activity.

The N-aroyl N'-phenyl ureas of the present invention are more active and have a broader spectrum of effectiveness than the benzoylurea insecticides currently available.

The novel compounds of this invention are compounds having the formula

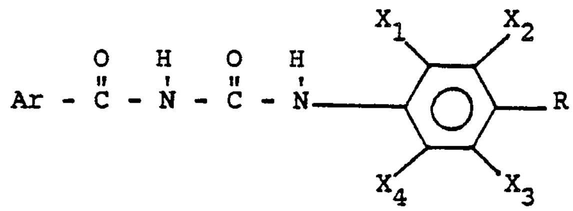

wherein Ar is a substituted phenyl radical wherein the substituents are chloro or fluoro with the proviso that at least one substituent is positioned ortho to the carbonyl group; $X_1$, $X_2$, $X_3$ and $X_4$ are individually H, halogen, $C_1$—$C_3$ alkyl, $C_1$—$C_3$ alkoxy, $C_1$—$C_3$ alkylcarbonyl, $C_1$—$C_3$ alkoxycarbonyl, $C_1$—$C_3$ alkylthio with the provisos that at least two of $X_1$, $X_2$, $X_3$ or $X_4$ are other than H and (a) when $X_1$ and $X_3$ are H, $X_2$ and $X_4$ cannot both be halogen and (b) when $X_2$ and $X_3$ are H, $X_1$ and $X_4$ cannot both be halogen; and R is a $C_1$—$C_4$ haloalkyl, $C_1$—$C_4$ haloalkoxy or $C_1$—$C_4$ haloalkylthio group.

These novel compounds can be prepared by methods analogous to those known in the art, e.g., as taught in U.S. Patent 4,139,636.

The invention also provides insecticidal compositions comprising an insecticidally effective amount of the above described N-aroyl N'-phenyl ureas in admixture with a suitable carrier or adjuvant therefore and a method for killing and/or controlling insects which comprises applying the active compound, alone or in admixture with a carrier, to the insects, the insect larvae or their habitats.

The preferred compounds of this invention are those having the formula

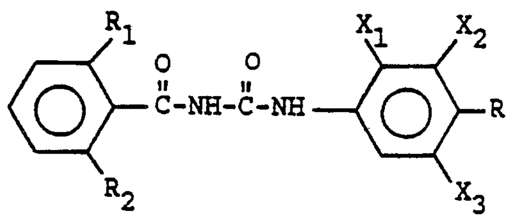

where $R_1$ is F or Cl; $R_2$ is F, Cl or H; $X_1$ is H, Cl or $CH_3$; $X_2$ and $X_3$ are H, Cl, $CH_3$ or $OCH_3$ and R is $CF_3$, $OCF_3$, $OCF_2CHF_2$, $OCF_2CHClF$ or $OCF_2CHFBr$. Most preferably, $R_1$ and $R_2$ are both fluorine, $X_2$ and $X_3$ are Cl or $CH_3$ and R is $OCF_2CHClF$, $OCF_2CHF_2$ or $OCF_2CHFBr$. Particularly preferred compounds are those wherein $X_1$ and $X_2$ are Cl and $X_3$ is H; $X_1$ and $X_3$ are $CH_3$ and $X_2$ is H; and $X_2$ and $X_3$ are $CH_3$ or Cl and $X_1$ is H.

The compounds of the present invention are normally crystalline solids of low solubility in water and of moderate solubility in many organic solvents. The compounds have low phytotoxicity and have exceptional activity in the control of various undesirable agricultural, household and veterinary insect pests.

Representative of the various insects which can be controlled by the active compounds of the present invention are members of the orders Lepidoptera, Coleoptera, Diptera, Orthoptera, Homoptera, Thysanoptera and Acarina. They are active against normally sensitive and resistant species at some stages of development. Examples of insect pests comprising the above include the tobacco budworm (*Heliothis virescens*), the beet armyworm (*Spodoptera exigua*), the Egyptian cotton leafworm (*Spodoptera littoralis*), the American bollworm (*Heliothis armigera*), the diamond-back moth (*Plutella maculipennis*), the gypsy moth (*Lymantria dispar*), the cutworm (*Agrotis segetum*), the Mediterranean flour moth (*Ephestia kuehniella*), the Colorado potato beetle (*Leptinotarsa decimlineata*), the mustard beetle (*Phaedon cochleariae*), the cotton boll weevil (*Anthonomus grandis*), the Mexican bean beetle (*Epilachna varivestis*), the khapra beetle (*Trogoderma granarium*), the housefly (*Musca domestica*), the lesser housefly (*Fannia canicularis*), the Mediterranean fruit fly (*Ceratitis capitata*), the black blow fly (*Phormia regina*), the cabbage rootfly (*Hylemya brassicae*), the yellow fever mosquito (*Aedes aegypti*), the malaria mosquito (*Anopheles stephensi*), the desert locust (*Schistocerca gregaria*), the migratory locust (*Locusta migratoria*), the German cockroach (*Blattells germanica*), the American cockroach (*Periplaneta americana*), the pear psylla (*Psylla pyricola*), the onion thrips (*Thrips tabaci*), and the citrus rust mite (*Phyllocoptruta oleivora*).

The compounds are highly active and can be employed to kill insects outright and/or to prevent adult emergence from juvenile forms of the insect. In such applications, the insect to be controlled and/or its habitat is contacted or treated with an insecticidal amount of one or more of the compounds of the present invention. The compounds may be administered orally to warm blooded animals from which they are excreted unchanged and they effectively combat the larvae of certain feces inhabiting insects, e.g., the face fly, horn fly and buffalo fly.

For all such uses, these compounds can be employed in unmodified form. However, the present invention embraces the use of an insecticidally-effective amount of the active ingredients in composition form with a material known in the art as an adjuvant or carrier.

Thus, for example, compositions employing one or a combination of these active ingredients can be in the form of a liquid or a dust; and the adjuvant employed can be any one of a plurality of materials including aromatic solvents, petroleum distillates, water or other liquid carriers, propellant substances, surface-active dispersing agents, light absorbers and finely-divided carrier solids.

The exact concentration of one or a combination of the compounds of the present invention in a composition thereof with an adjuvant therefore can vary; it is only necessary that one or a combination of the compounds be present in a sufficient amount so as to make possible the application of an insecticidally-effective or inactivating dosage.

Generally, for practical applications, one or a combination of these active ingredients can be broadly applied to the insect larvae or their habitat in compositions containing from about 0.0001 to about 98 percent by weight, preferably 5 to 50 percent by weight, of the compounds.

The invention is further illustrated by the following examples.

Example 1

N-(((3,5-Dichloro-4-(2,2-dichloro-1,1-difluoroethoxy)phenyl)amino)carbonyl)-2,6-difluoro-N-methylbenzamide

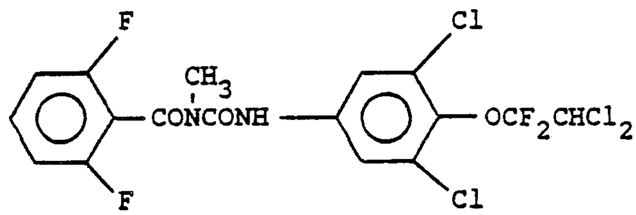

N - (((3,5 - Dichloro - 4 - (2,2 - dichloro - 1,1 - difluoroethoxy)phenyl)amino)carbonyl) - 2,6 - difluorobenzamide (9.9 g, 0.02 mole) and KOH (1.4 g, 87 percent purity, 0.02 mole) were added to 50 ml dimethylformamide with stirring, giving a clear solution. Iodomethane (3.2 g, 0.022 mole) was added and the resulting solution was stirred at room temperature for about 24 hours. The precipitated product was collected by suction filtration and dried. The product was purified by recrystallization from isopropanol. The purified product was a white solid melting at 153—155°C. NMR spectral features support the structural assignment.

| | | | |
|---|---|---|---|
| Elemental Analysis: Calcd: | C, 40.18; | H, 1.98; | N, 5.51 |
| Found: | C, 40.3; | H, 2.10; | N, 5.43 |

Example 2

(A) Preparation of 2,6-diethyl-4-nitrophenol

A solution of 45 g (0.3 mole) of 2,6-diethylphenol in 300 ml of chloroform was prepared in a 500 ml, 3-necked round bottom flask fitted with a mechanical stirrer, thermometer and dropping funnel and 27 g (0.3 mole) of 70 percent $HNO_3$ was added dropwise with stirring and cooling at 25°—30°C in 1/2 hour. Stirring at room temperature was continued for 5 hours. The mixture was washed with 100 ml of water and the solvent removed in a rotary evaporator. A dark red oil (86 g) was obtained which partially crystallized on standing. This was cooled in an ice water bath and the resulting solid removed by suction filtration. The cake (A) was washed with cold chloroform and dried at room temperature (19 g). NMR indicated the product was 2,6-diethyl-4-nitrophenol (93 percent pure). The solvent was removed from the filtrate in a rotary evaporator leaving 43 g of residue (B). Gas chromatography indicated 47 percent starting material, 22 percent desired product, plus two higher products. (B) was dissolved in 200 ml chloroform, returned to the reactor and 13 ml of $HNO_3$ was added dropwise with stirring in 5 minutes. The temperature rose from 23° to 30°C. Stirring at room temperature was continued for 5 hours and the mixture worked up as above. 62 Grams of a dark red oil (C) was obtained which was placed in the refrigerator overnight. Suction filtration provided 5 grams of solid (D).

(B) Preparation of 4-amino-2,6-diethylphenol·HCl

Cake (A) and solid (D) were combined (24 g) and placed in a Pyrex bottle with 200 ml of 95 percent ethanol and 2 g of 5 percent palladium on carbon catalyst and hydrogenated in a Parr apparatus at

30—50 psig for 2.75 hours using a heat lamp. Hydrogenation almost stopped when 16 psig hydrogen absorbed. Two more grams of catalyst were added and hydrogenation was continued for 1.33 hours when hydrogenation stopped. An additional 20 psig hydrogen absorbed, although there was a small leak in the apparatus. The mixture was cooled and suction filtered to remove the catalyst which was washed with some ethanol. The ethanol was removed from the filtrate in a rotary evaporator. The residue, a brown, oily, low-melting solid, weighed 28 g. The solid was dissolved in 250 ml of methylene dichloride which was then saturated with dry HCl while cooling in an ice water bath. After filtering with suction, the resulting cake was washed with cold methylene dichloride and dried at room temperature. 16 Grams of a tan solid was obtained, m.p. 230°—235°C. The yield was 66 percent and the structure was confirmed by NMR.

(C) Preparation of 4-(2-chloro-1,1,2-trifluoroethoxy)-3,5-diethylbenzenamine

The above tan solid (16 g) was placed in a 500 ml round bottom flask fitted with a mechanical stirrer, thermometer, reflux condenser and sparger, along with 150 ml of dimethylformamide and 9.0 g (0.16 mole) of KOH pellets which were ground to powder. The mixture was heated while stirring to 90°C and 12 g (0.1 mole) of chlorotrifluoroethylene was bubbled in at 90°—100°C in 35 minutes. Stirring was continued at about 90°C for 5 minutes, the mixture was cooled with an ice water bath and 300 ml of cold water was added. The mixture was extracted with 200 ml of methylene dichloride and then 100 ml of methylene dichloride. The extracts were combined, washed with 250 ml of water and the methylene dichloride removed in a rotary evaporator. The residue was distilled through a short column. The second cut (120°—130°C/2 mm) was a light tan viscous liquid weighing 9 g. The structure was confirmed by NMR and the yield was 40 percent.

(D) Preparation of N-(((4-(2-chloro-1,1,2-trifluoroethoxy)-3,5-diethylphenyl)amino)carbonyl)-2,6-difluorobenzamide

The light tan liquid prepared above (9 g, 0.03 mole) was dissolved in 150 ml toluene in a 500 ml 3-necked round bottom flask fitted with a mechanical stirrer, thermometer and reflux condenser and 7.3 g (0.04 mole) of 2,6-difluorobenzoyl isocyanate was added while stirring. The temperature rose from 21° to 26°C. The mixture was heated at reflux for 1 hour and filtered hot through fluted filter paper to remove a trace of dirt. The filtrate was cooled in an ice water bath and the upper solvent layer decanted off about 1 g of a brown gum. The toluene was removed in a rotary evaporator leaving a residue of 17 g of a dark brown viscous oil to which 25 ml of isopropanol was added. Upon heating to boiling, a clear solution developed which was cooled in an ice water bath. The resulting crystals were removed by suction filtration, the filter cake was washed with cold isopropanol and dried at room temperature. A light tan solid (6.5 g, m.p. 122°—124°C) was obtained. The structure was confirmed by NMR.

| | | | |
|---|---|---|---|
| Analysis: Calcd: | C, 51.68; | H, 3.90; | 6.03 |
| Found: | C, 51.5; | H, 3.93; | N, 6.04. |

Employing the above procedures and appropriate starting materials, the following compounds were prepared:

N-(((3,5-Dichloro-4-(1,1,2,2-tetrafluoroethoxy)phenyl)amino)carbonyl)-2,6-difluorobenzamide

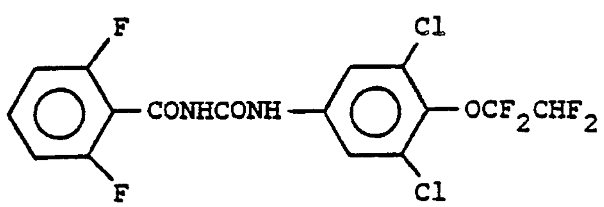

M.P. = 197°—199°C.

| | | | |
|---|---|---|---|
| Analysis: Calcd: | C, 41.67; | H, 1.75; | N, 6.08 |
| Found: | C, 41.8; | H, 1.92; | N, 6.06. |

N-(((3,5-Dichloro-4-(1,1,2,2-tetrafluoroethoxy)phenyl)amino)carbonyl)-2-chlorobenzamide

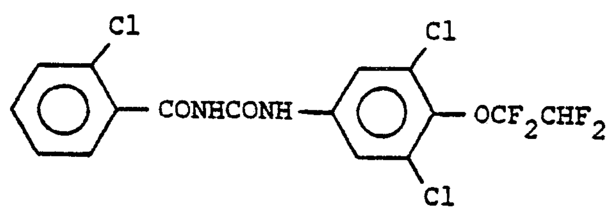

M.P. = 173°—175°C.

| | |
|---|---|
| Analysis: Calcd: | N, 6.10 |
| Found: | N, 6.34 |

N-(((3,5-Dichloro-4-(2,2-dichloro-1,1-difluoroethoxy)phenyl)amino)carbonyl)-2,6-difluorobenzamide

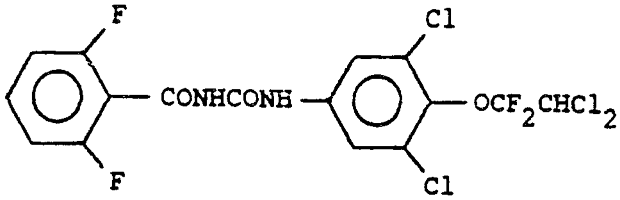

M.P. = 199°—201°C.
Analysis: Calcd: N, 5.67
Found: N, 5.66

N-(((3,5-Dichloro-4-(2,2-dichloro-1,1-difluoroethoxy)phenyl)amino)carbonyl)-2-chlorobenzamide

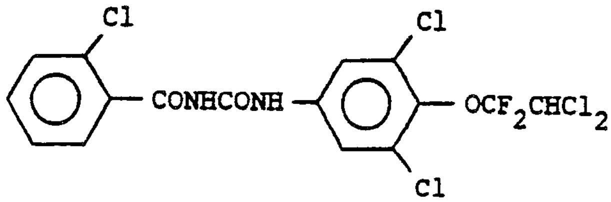

M.P. = 200°—202°C.
Analysis: Calcd: N, 5.69
Found: N, 5.60.

N-(((4-(2-Chloro-1,1,2-trifluoroethoxy)-2,5-dimethylphenyl)amino)carbonyl)-2-chlorobenzamide.
M.P. 157°—159°C.
Analysis: Calcd: C, 49.67; H, 3.47; N, 6.44
Found: C, 49.40; H, 3.36; N, 6.41.

N-(((4-(2-Chloro-1,1,2-trifluoroethoxy)-2,5-dimethylphenyl)amino)carbonyl)-2-chloro-6-fluorobenzamide.
M.P. 145°—147°C.
Analysis: Calcd: C, 47.70; H, 3.11; N, 6.18
Found: C, 47.70; H, 3.09; N, 6.22.

N-(((4-(1,1,2,2-Tetrafluoroethoxy)-3,5-dimethylphenyl)amino)carbonyl)-2,6-difluorobenzamide.
M.P. 190°—192°C.
Analysis: Calcd: C, 51.43; H, 3.36; N, 6.67
Found: C, 51.30; H, 3.45; N, 6.59.

N-(((4-(1,1,2,2-Tetrafluoroethoxy)-3,5-dimethylphenyl)amino)carbonyl)-2-chlorobenzamide
M.P. 165°—167°C
Analysis: Calcd: C, 51.62; H, 3.61; N, 6.69
Found: C, 51.9; H, 3.60; H, 6.67.

N-(((4-(2-Chloro-1,1,2-trifluoroethoxy)-3,5-dimethylphenyl)amino)carbonyl)-2,6-difluorobenzamide.
M.P. 184°—186°C.
Analysis: Calcd: C, 49.50; H, 3.23; N, 6.42
Found: C, 49.60; H, 3.18; N, 6.38.

N-(((4-(2-Chloro-1,1,2-trifluoroethoxy)-3,5-dimethylphenyl)amino)carbonyl)-2-chlorobenzamide
M.P. 152°—154°C
Analysis: Calcd: C, 49.67; H, 3.47; N, 6.44
Found: C, 49.70; H, 3.45; N, 6.34.

N-(((4-(2-Chloro-1,1,2-trifluoroethoxy)-2,5-dimethylphenyl)amino)carbonyl)-2,6-difluorobenzamide.
M.P. 143°—145°C.
Analysis: Calcd: C, 49.50; H, 3.23; N, 6.42
Found: C, 49.50; H, 3.24; N, 6.34.

In addition to the above described preparative methods, many of the compounds of this invention can be made by reacting a compound having the formula

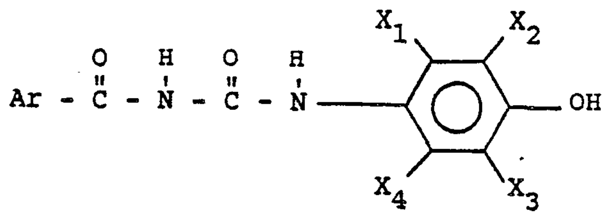

wherein all of the substituents are as above defined with a haloalkene as is known in the art.

The biological activity of several of these compounds was determined. In the beet armyworm test, cotton leaves were dipped in aqueous suspensions of the chemicals, dried, excised and placed into petri dishes with five second-instar beet armyworm (*Spodoptera exigua*) larvae. Mortality counts were made five days later. The tobacco budworm test was the same except that five tobacco budworm (*Heliothis virescens*) larvae were placed onto the treated leaves. The results are summarized below:

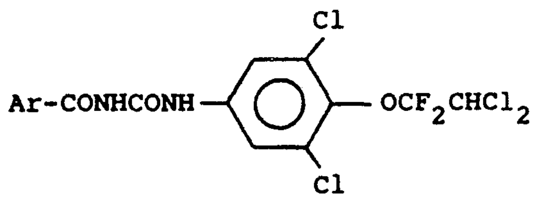

| | | Percent Control at Indicated Dosage, ppm | | | | | |
|---|---|---|---|---|---|---|---|
| | | Beet Army Worm Test (1) | | | Tobacco Budworm Test (2) | | |
| Compound | Ar | 400 | 100 | 25 | 400 | 100 | 25 |
| A | 2,6-difluorophenyl | 100 | 100 | 100 | 100 | 100 | 80 |
| B | 2-chlorophenyl | 100 | 100 | 100 | 100 | 80 | 40 |

(1) Untreated check mortality 0%.
(2) Untreated check mortality 27%.

Employing the above described preparative and testing methods, the compounds listed in the following table were prepared and tested. The test results, $LD_{90}$ ppm, indicate the dosage necessary to obtain 90 percent kill of the indicated insect.

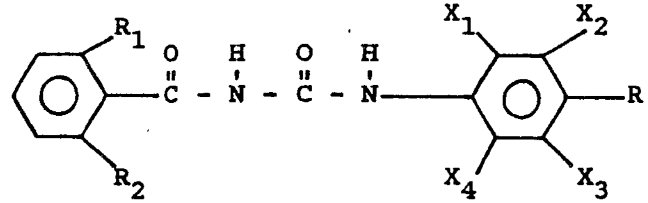

| Ex. No. | $R_1$ | $R_2$ | Y | $X_1$ | $X_2$ | $X_3$ | $X_4$ | R | Melting Point °C | $LD_{90}$, ppm Beet Armyworm | $LD_{90}$, ppm Tobacco Budworm |
|---|---|---|---|---|---|---|---|---|---|---|---|
| 1 | F | F | O | H | Cl | Cl | H | $OCF_2CHF_2$ | 197—199 | 1.6 | 1.6 |
| 2 | F | F | O | H | Cl | Cl | H | $OCF_2CHFCl$ | 176—178 | 1.2 | 1.2 |
| 3 | F | F | O | H | Cl | Cl | H | $OCF_2CHFBr$ | 170—172 | 1.2 | 2.5 |
| 4 | F | F | O | H | Cl | Cl | H | $OCF_2CHFI$ | 170—172 | | 12.5 |
| 5 | F | F | O | H | Cl | Cl | H | $OCF_2CHCl_2$ | 197—199 | 5.0 | 25.0 |
| 6 | F | F | O | H | Cl | Cl | H | $OCF_2CH_2Cl$ | 188—190 | 100.0 | 100.0 |
| 7 | F | F | O | H | Cl | Cl | H | $OCF_2CHBr_2$ | 215—217 | 25.0 | 400.0 |
| 8 | F | F | O | H | Cl | Cl | H | OCFClCHFCl | 203—205 | 1.25 | 25.0 |
| 9 | F | F | O | H | Cl | Cl | H | $OCF_3$ | 218—220 | 0.6 | 12.5 |
| 10 | F | F | O | H | Cl | Cl | H | $OCF_2CF_3$ | 224—227 | 12.5 | 50.0 |
| 11 | F | F | O | H | Cl | Cl | H | $SCF_2CHFCl$ | 250—260 | 200.0 | >400.0 |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 12 | F | F | O | H | Cl | Cl | H | $SCF_2CHF_2$ | 166—168 | 3.1 | 12.5 |
| 13 | Cl | H | O | H | Cl | Cl | H | $OCF_2CHFCl$ | 178—180 | 5.0 | 6.2 |
| 14 | Cl | H | O | H | Cl | Cl | H | $OCF_2CHF_2$ | 173—175 | 100.0 | 25.0 |
| 15 | Cl | H | O | H | Cl | Cl | H | $OCF_2CHFBr$ | 183—185 | 2.5 | 10.0 |
| 16 | Cl | H | O | H | Cl | Cl | H | $OCF_2CHFI$ | 171—174 | | 50.0 |
| 17 | Cl | H | O | H | Cl | Cl | H | $OCF_2CHCl_2$ | 200—202 | 6.2 | 200.0 |
| 18 | Cl | H | O | H | Cl | Cl | H | $OCF_2CHBr_2$ | 217—219 | 25.0 | 400.0 |
| 19 | Cl | H | O | H | Cl | Cl | H | $OCF_2$ | 203—205 | 2.5 | 12.5 |
| 20 | F | H | O | H | Cl | Cl | H | $SCF_2CHFCl$ | 245—250 | >400.0 | >400.0 |
| 21 | F | H | O | H | Cl | Cl | H | $OCF_2CHFCl$ | 165—167 | 100.0 | 25.0 |
| 22 | F | Cl | O | H | Cl | Cl | H | $OCF_2CHF_2$ | | | |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 23 | F | Cl | O | H | Cl | Cl | H | $OCF_2CHFCl$ | 205—207 | 2.5 | 5.0 |
| 24 | F | Cl | O | H | Cl | Cl | H | $OCF_2CHFBr$ | 184—186 | 2.5 | 5.0 |
| 25 | Cl | Cl | O | H | Cl | Cl | H | $OCF_2CHF_2$ | 217—219 | 25.0 | 25.0 |
| 26 | Cl | Cl | O | H | Cl | Cl | H | $OCF_2CHFCl$ | 221—223 | 6.2 | 25.0 |
| 27 | Cl | Cl | O | H | Cl | Cl | H | $OCF_2CHFBr$ | 200—202 | 100.0 | 12.5 |
| 28 | Cl | Cl | O | H | Cl | Cl | H | $SCF_2CHF_2$ | 213—215 | 50.0 | 200.0 |
| 29 | F | F | O | H | Br | Br | H | $OCF_2CHF_2$ | 201—203 | 3.7 | 10.0 |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 30 | F | F | O | Cl | Cl | H | H | $OCF_2CHFCl$ | 180—182 | 0.6 | 2.5 |
| 31 | F | F | O | Cl | Cl | H | H | $OCF_2CHF_2$ | 166—169 | | 12.5 |
| 32 | F | F | O | Cl | Cl | H | H | $OCF_2CHFBr$ | 169—171 | 1.6 | 1.6 |
| 33 | F | F | O | $CH_3$ | H | H | H | $OCF_2CHFCl$ | 169—171 | | |
| 34 | Cl | H | O | $CH_3$ | $CH_3$ | H | H | $OCF_2CHFCl$ | 150—152 | | |
| 35 | F | F | O | $CH_3$ | H | $CH_3$ | H | $OCF_2CHF_2$ | | | |
| 36 | F | F | O | $CH_3$ | H | $CH_3$ | H | $OCF_2CHFCl$ | 143—145 | | 3.1 |
| 37 | F | F | O | $CH_3$ | H | $CH_3$ | H | $OCF_2CHFBr$ | | | |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 38 | Cl | H | O | $CH_3$ | H | $CH_3$ | H | $OCF_2CHFCl$ | 157—159 | | 12.5 |
| 39 | F | Cl | O | $CH_3$ | H | $CH_3$ | H | $OCF_2CHFCl$ | 145—147 | | 12.5 |
| 40 | F | F | O | $CH_3$ | H | H | $CH_3$ | $OCF_2CHFCl$ | 191—193 | | |
| 41 | F | F | O | H | $CH_3$ | $CH_3$ | H | $OCF_2CHF_2$ | 190—192 | | 3.1 |
| 42 | F | F | O | H | $CH_3$ | $CH_3$ | H | $OCF_2CHFCl$ | 184—186 | 1.2 | 1.2 |
| 43 | F | F | O | H | $CH_3$ | $CH_3$ | H | $OCF_2CHFBr$ | 176—178 | 3.1 | 3.1 |
| 44 | Cl | H | O | H | $CH_3$ | $CH_3$ | H | $OCF_2CHF_2$ | 165—167 | 12.5 | 12.5 |
| 45 | Cl | H | O | H | $CH_3$ | $CH_3$ | H | $OCF_2CHFCl$ | 152—154 | 5.0 | 12.5 |
| 46 | Cl | H | O | H | $CH_3$ | $CH_3$ | H | $OCF_2CHFBr$ | 147—149 | 6.2 | 25.0 |
| 47 | F | Cl | O | H | $CH_3$ | $CH_3$ | H | $OCF_2CHF_2$ | | | |
| 48 | F | Cl | O | H | $CH_3$ | $CH_3$ | H | $OCF_2CHFCl$ | | | |
| 49 | F | Cl | O | H | $CH_3$ | $CH_3$ | H | $OCF_2CHFBr$ | | | |
| 50 | F | F | O | H | $C_2H_5$ | $C_2H_5$ | H | $OCF_2CHFCl$ | 122—124 | 25.0 | 25.0 |
| 51 | F | F | O | $CH_3$ | $CH_3$ | $CH_3$ | H | $OCF_2CHF_2$ | | | |
| 52 | F | F | O | $CH_3$ | $CH_3$ | $CH_3$ | H | $OCF_2CHFCl$ | 152—154 | 3.1 | 3.1 |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 53 | F | F | O | $CH_3$ | $CH_3$ | $CH_3$ | H | $OCF_2CHFBr$ | | | |
| 54 | F | F | O | $CH_3$ | $CH_3$ | $CH_3$ | $CH_3$ | $OCF_2CHF_2$ | | | |
| 55 | F | F | O | H | $OCH_3$ | $OCH_3$ | H | $OCF_2CHFCl$ | 142—144 | 10.0 | 10.0 |
| 56 | D | F | O | $CH_3$ | H | Cl | H | $OCF_2CHF_2$ | | | |
| 57 | F | F | O | $CH_3$ | H | Cl | H | $OCF_2CHFCl$ | 178—180 | | |
| 58 | Cl | H | O | $CH_3$ | H | Cl | H | $OCF_2CHFCl$ | | | |
| 59 | Cl | H | O | H | Cl | $CH_3$ | H | $OCF_2CHFCl$ | | | |
| 60 | F | F | O | H | Cl | $CH_3$ | H | $OCF_2CHFCl$ | 175—177 | | |
| 61 | F | F | O | $CH_3$ | Cl | Cl | H | $OCF_2CHF_2$ | | | |
| 62 | F | F | O | $CH_3$ | Cl | Cl | H | $OCF_2CHFCl$ | 190—192 | | |
| 63 | Cl | H | O | $CH_3$ | Cl | Cl | H | $OCF_2CHFCl$ | 167—169 | | |
| 64 | F | F | O | $CH_3$ | Cl | Cl | $CH_3$ | $OCF_2CHFCl$ | 240—241 | | |
| 65 | F | Cl | O | $CH_3$ | Cl | Cl | $CH_3$ | $OCF_2CHFCl$ | 228—229 | | |

| Ex. No. | $R_1$ | $R_2$ | Y | $X_1$ | $X_2$ | $X_3$ | $X_4$ | R | Melting Point °C | $LD_{90}$, ppm Beet Armyworm | Tobacco Budworm |
|---|---|---|---|---|---|---|---|---|---|---|---|
| 66 | F | F | O | H | $\overset{O}{\overset{\|}{C}}CH_3$ | $CH_3$ | H | $OCF_2CHFCl$ | 163—165 | | |
| 67 | F | F | O | H | $\overset{O}{\overset{\|}{C}}OCH_3$ | $CH_3$ | H | $OCF_2CHCl_2$ | 153—155 | | |
| 68 | Cl | H | O | H | $\overset{O}{\overset{\|}{C}}OCH_3$ | $CH_3$ | H | $OCF_2CHCl_2$ | 143—145 | | |
| 69 | F | F | O | H | $NO_2$ | H | H | $OCF_2CHFCl$ | 227—228 | | |
| 70 | Cl | H | O | H | $NO_2$ | H | H | $OCF_2CHFCl$ | 206—208 | | |
| 71 | F | F | O | Cl | Cl | Cl | H | $OCF_2CHCl_2$ | 181—183 | 5.0 | 5.0 |
| 72 | F | F | O | Cl | Cl | Cl | H | $OCF_2CHF_2$ | | | |
| 73 | F | F | O | Cl | Cl | Cl | H | $OCF_2CHFCl$ | | | |
| 74 | F | F | O | Cl | Cl | Cl | H | $OCF_2CHFBr$ | | | |

In further embodiments, the compounds of the present invention or compositions containing the same, can be advantageously employed in combination with one or more additional pesticidal compounds. Such additional pesticidal compounds may be insecticides, nematocides, acaricides, herbicides, fungicides or bactericides that are compatible with the compounds of the present invention in the medium selected for application and not antagonistic to the activity of the present compounds. Accordingly, in such embodiments, the pesticidal compound is employed as a supplemental toxicant for the same or for a different pesticidal use, or as an additament. The compounds in combination can generally be present in the ratio of from 1 to 100 parts of the compound of the present invention with from 100 to 1 parts of the additional compound(s).

The compounds of this invention are, or tend to be, slow acting, i.e., they disrupt the molting of the insect, thereby killing it. As a result, some time can pass before the insects are killed. Accordingly, an increased benefit can be obtained by combining the compounds of this invention with quicker acting insecticides such as, for example, organophosphorus compounds, carbamates and pyrethroids. Because of this different mode of action, the compounds of this invention kill or control insects which have, or may be developing, resistance to the more common insecticides which have, or may be developing, resistance to the more common insecticides and thus they inhibit or delay the development of resistance to such insecticides.

Various modifications may be made in the present invention without departing from the spirit or scope thereof and it is understood that we limit ourselves only as defined in the appended claims.

**Claims**

1. A compound having the formula

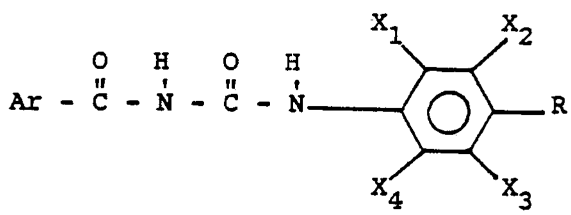

wherein Ar is a substituted phenyl radical wherein the substituents are chloro or fluoro with the proviso that at least one substituent is positioned ortho to the carbonyl group; $X_1$, $X_2$, $X_3$ and $X_4$ are individually H, halogen, $C_1$—$C_3$ alkyl, $C_1$—$C_3$ alkoxy, $C_{1-3}$ alkylcarbonyl, $C_{1-3}$ alkoxycarbonyl, $C_1$—$C_3$ alkylthio with the provisos that at least two of $X_1$, $X_2$, $X_3$ or $X_4$ are other than H and (a) when $X_1$ and $X_3$ are H, $X_2$ and $X_4$ cannot both be halogen and (b) when $X_2$ and $X_3$ are H, $X_1$ and $X_4$ cannot both be halogen; and R is a $C_1$—$C_4$ haloalkyl, $C_{1-4}$ haloalkoxy or $C_{1-4}$ haloalkylthio group.

2. Compound of Claim 1 having the formula

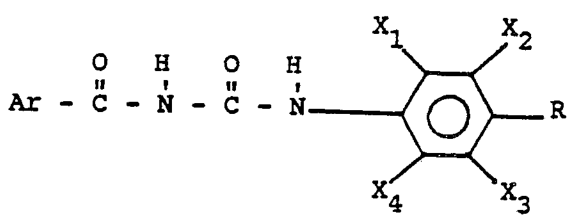

wherein $X_1$ and $X_4$ are hydrogen and $X_2$ and $X_3$ are halogen.

3. Compound of Claim 2 having the formula

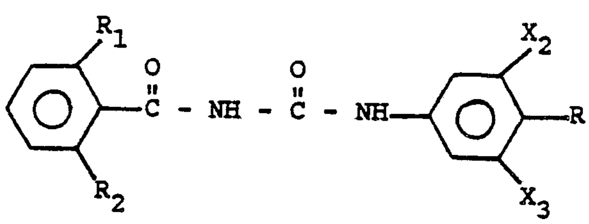

wherein $R_1$ is F or Cl and $R_2$ is H, F or Cl.

4. Compound of Claim 3 wherein $X_2$ and $X_3$ are independently F, Cl or Br and R is $OCF_3$, $OCF_2CHCl_2$, $OCF_2CHF_2$, $OCF_2CHFCl$, $OCF_2CHFBr$, $OCF_2CHFI$, $OCFClCHFCl$ or $SCF_2CHF_2$.

5. Compound of Claim 4 wherein $R_1$ and $R_2$ are both F, $X_2$ and $X_3$ are both Cl, and R is $OCF_2CHF_2$.

6. Compound of Claim 4 wherein $R_1$ and $R_2$ are both F, $X_2$ and $X_3$ are both Cl, and R is $OCF_2CHFCl$.

7. Compound of Claim 4 wherein $R_1$ and $R_2$ are both F, $X_2$ and $X_3$ are both Cl, and R is $OCF_2CHFBr$.

8. Compound of Claim 4 wherein $R_1$ is Cl, $R_2$ is H, $X_2$ and $X_3$ are both Cl, and R is $OCF_2CHF_2$, $OCF_2CHFCl$ or $OCF_2CHFBr$.

9. Compound of Claim 4 wherein $R_1$ is Cl, $R_2$ is F, $X_2$ and $X_3$ are both Cl, and R is $OCF_2CHF_2$, $OCF_2CHFCl$ or $OCF_2CHFBr$.

10. Compound of Claim 1 having the formula

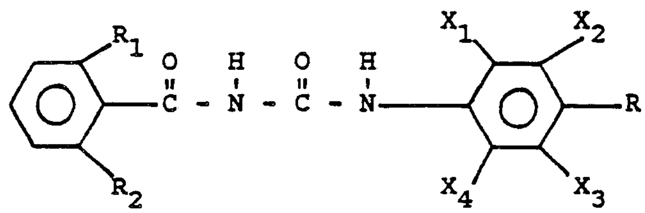

wherein $R_1$ is F or Cl, $R_2$ is H, F or Cl, $X_1$, $X_2$, $X_3$ and $X_4$ are individually H, halogen, alkyl or alkoxy and R is a $C_1$—$C_4$ haloalkyl, haloalkoxy or haloalkylthio group.

11. Compound of Claim 10 wherein $X_1$, $X_2$, $X_3$ and $X_4$ are individually H, Cl, F, Br, $CH_3$ or $OCH_3$ and R is $CF_3$, $OCF_3$, $OCF_2CHCl_2$, $OCF_2CHF_2$, $OCF_2CHFCl$, $OCF_2CHFBr$, $OCF_2CHFI$, $OCFClCHFCl$ or $SCF_2CHF_2$.

12. Compound of Claim 11 having the formula

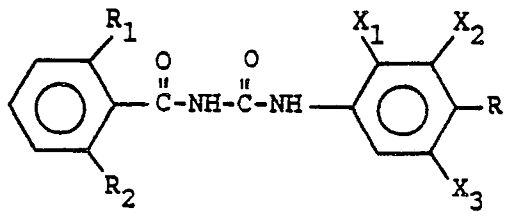

wherein $R_1$ is F or Cl, $R_2$ is H, F or Cl, $X_1$ is H, Cl or $CH_3$, $X_2$ is H, Cl, $CH_3$ or $OCH_3$, $X_3$ is H, Cl, $CH_3$ or $OCH_3$ and R is $OCF_2CHF_2$, $OCF_2CHFCl$ or $OCF_2CHFBr$.

13. Compound of Claim 12 wherein $R_1$ and $R_2$ are both F, $X_1$ and $X_2$ are both Cl, $X_3$ is H and R is $OCF_2CHF_2$, $OCF_2CHFCl$ or $OCF_2CHFBr$.

14. Compound of Claim 12 wherein $R_1$ and $R_2$ are both F, $X_1$ is H, $X_2$ and $X_3$ are both $CH_3$ and R is $OCF_2CHFCl$.

15. Compound of Claim 12 wherein $R_1$ and $R_2$ are both F, $X_1$ is H, $X_2$ and $X_3$ are both $CH_3$ and R is $OCF_2CHF_2$.

16. Compound of Claim 12 wherein $R_1$ and $R_2$ are both F, $X_1$ is H, $X_2$ and $X_3$ are both $CH_3$ and R is $OCF_2CHFBr$.

17. Compound of Claim 12 wherein $R_1$ is Cl, $R_2$ is F, $X_1$ is H, $X_2$ and $X_3$ are both $CH_3$ and R is $OCF_2CHF_2$, $OCF_2CHFCl$ or $OCF_2CHFBr$.

18. Compound of Claim 12 wherein $R_1$ and $R_2$ are both F, $X_1$ and $X_3$ are both $CH_3$, $X_2$ is H and R is $OCF_2CHF_2$, $OCF_2CHFCl$ or $OCF_2CHFBr$.

19. Compound of Claim 12 wherein $R_1$ and $R_2$ are both F, $X_1$, $X_2$ and $X_3$ are $CH_3$ and R is $OCF_2CHF_2$, $OCF_2CHFCl$ or $OCF_2CHFBr$.

20. Compound of Claim 12 wherein $X_1$ is methyl and $X_2$ and $X_3$ are chloro.

21. A compound having the formula

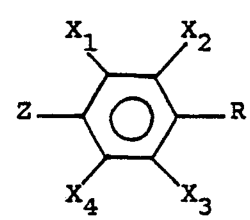

wherein $X_1$, $X_2$, $X_3$ and $X_4$ are independently H, halogen, $C_1$—$C_3$ alkyl, $C_1$—$C_3$ alkoxy, $C_{1-3}$ alkylcarbonyl, $C_{1-3}$ alkoxycarbonyl, $C_1$—$C_3$ alkylthio with the provisos that at least two of $X_1$, $X_2$, $X_3$ or $X_4$ are other than H and (a) when $X_1$ and $X_3$ are H, $X_2$ and $X_4$ cannot both be halogen and (b) when $X_2$ and $X_3$ are H, $X_1$ and $X_4$ cannot both be halogen; R is a $C_1$—$C_4$ haloalkyl, $C_{1-4}$ haloalkoxy or $C_{1-4}$ haloalkylthio group and Z is $NH_2$,

$$-N=C=O,\quad -NH-\overset{\overset{O}{\|}}{C}-NH_2,\quad N=C=S \quad \text{or} \quad -NH-\overset{\overset{S}{\|}}{C}-NH_2.$$

22. Compound of Claim 21 wherein $X_1$ and $X_4$ are hydrogen and $X_2$ and $X_3$ are halogen.

23. Compound of Claim 22 wherein $X_2$ and $X_3$ are independently F, Cl or Br and R is $OCF_3$, $OCF_2CHCl_2$, $OCF_2CHF_2$, $OCF_2CHFCl$, $OCF_2CHFBr$, $OCF_2CHFI$, $OCFClCHFCl$ or $SCF_2CHF_2$.

24. Compound of Claim 23 wherein $X_2$ and $X_3$ are both Cl and R is $OCF_2CHF_2$.

25. Compound of Claim 23 wherein $X_2$ and $X_3$ are both Cl and R is $OCF_2CHFCl$.

26. Compound of Claim 23 wherein $X_2$ and $X_3$ are both Cl and R is $OCF_2CHFBr$.

27. Compound of Claim 21 wherein $X_1$ and $X_2$ are both Cl, $X_3$ and $X_4$ are H and R is $OCF_2CHF_2$, $OCF_2CHFCl$ and $OCF_2CHFBr$.

28. Compound of Claim 21 wherein $X_1$ and $X_4$ are both H, $X_2$ and $X_3$ are both $CH_3$ and R is $OCF_2CHFCl$.

29. Compound of Claim 21 wherein $X_1$ and $X_4$ are H, $X_2$ and $X_3$ are both $CH_3$ and R is $OCF_2CHF_2$.

30. Compound of Claim 21 wherein $X_1$ and $X_4$ are H, $X_2$ and $X_3$ are both $CH_3$ and R is $OCF_2CHFBr$.

31. Compound of Claim 21 wherein $X_1$ and $X_3$ are both $CH_3$, $X_2$ and $X_4$ are H and R is $OCF_2CHF_2$, $OCF_2CHFCl$ or $OCF_2CHFBr$.

32. Compound of Claim 21 wherein $X_1$, $X_2$ and $X_3$ are $CH_3$, $X_4$ is H and R is $OCF_2CHF_2$, $OCF_2CHFCl$ or $OCF_2CHFBr$.

33. A method for killing and/or controlling insects which comprises applying to the insects, the insect larvae or their habitats an insecticidally effective amount of a compound having the formula

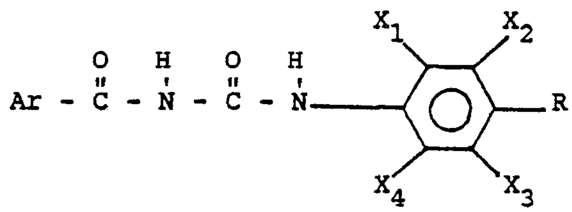

wherein Ar is a substituted phenyl radical wherein the substituents are chloro or fluoro, with the proviso that at least one substituent is positioned ortho to the carbonyl group; $X_1$, $X_2$, $X_3$ and $X_4$ are individually H, halogen, $C_1$—$C_3$ alkyl, $C_1$—$C_3$ alkoxy, $C_{1-3}$ alkylcarbonyl, $C_{1-3}$ alkoxycarbonyl, $C_1$—$C_3$ alkylthio with the provisos that at least two of $X_1$, $X_2$, $X_3$ or $X_4$ are other than H and (a) when $X_1$ and $X_3$ are H, $X_2$ and $X_4$ cannot both be halogen and (b) when $X_2$ and $X_3$ are H, $X_1$ and $X_4$ cannot both be halogen; and R is a $C_1$—$C_4$ haloalkyl, $C_{1-4}$ haloalkoxy or $C_{1-4}$ haloalkylthio group.

34. Method of Claim 33 wherein the compound employed has the formula

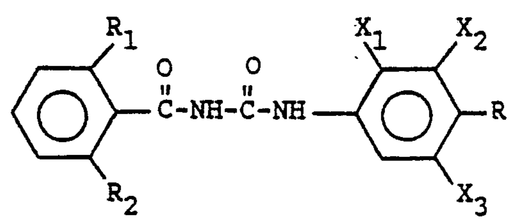

wherein $R_1$ is F or Cl; $R_2$ is H, F or Cl; $X_1$ is H, Cl or $CH_3$; $X_2$ and $X_3$ are H, Cl, $CH_3$ or $OCH_3$ and R is $OCF_3$, $OCF_2CHF_2$, $OCF_2CHFCl$, $OCF_2CHFBr$, $OCF_2CHFI$, $OCFClCHFCl$ or $SCF_2CHF_2$.

35. Method of Claim 34 wherein $X_2$ and $X_3$ are H, Cl or $CH_3$ and R is $OCF_2CHF_2$, $OCF_2CHFCl$ or $OCF_2CHFBr$.

36. Method of Claim 35 wherein $X_1$ and $X_2$ are both Cl, and $X_3$ is H.

37. Method of Claim 35 wherein $X_2$ and $X_3$ are both Cl and $X_1$ is H.

38. Method of Claim 35 wherein $X_1$ and $X_3$ are both $CH_3$, and $X_2$ is H.

39. Method of Claim 35 wherein $X_2$ and $X_3$ are both $CH_3$, and $X_1$ is H.

40. Method of Claim 35 wherein $X_1$, $X_2$ and $X_3$ are all $CH_3$.

41. A composition comprising an insecticidally effective amount of at least one compound having the formula

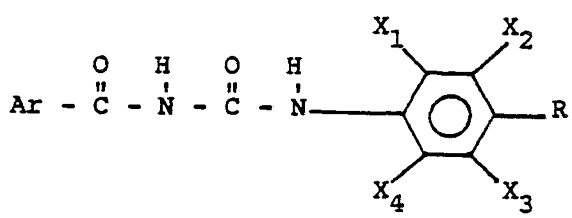

wherein Ar is a substituted phenyl radical wherein the substituents are chloro or fluoro, with the proviso that at least one substituent is positioned ortho to the carbonyl group; $X_1$, $X_2$, $X_3$ and $X_4$ are individually H, halogen, $C_1$—$C_3$ alkyl, $C_1$—$C_3$ alkoxy, $C_{1-3}$ alkylcarbonyl, $C_{1-3}$ alkoxycarbonyl, $C_1$—$C_3$ alkylthio with the

provisos that at least two of $X_1$, $X_2$, $X_3$ or $X_4$ are other than H and (a) when $X_1$ and $X_3$ are H, $X_2$ and $X_4$ cannot both be halogen and (b) when $X_2$ and $X_3$ are H, $X_1$ and $X_4$ cannot both be halogen; and R is a $C_1$—$C_4$ haloalkyl, $C_{1-4}$ haloalkoxy or $C_{1-4}$ haloalkylthio group.

42. Composition of Claim 41 wherein the compound has the formula

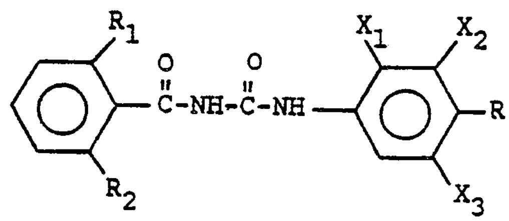

wherein $R_1$ is F or Cl; $R_2$ is H, F or Cl; $X_1$ is H, Cl or $CH_3$; $X_2$ and $X_3$ are H, Cl, $CH_3$ or $OCH_3$ and R is $OCF_3$, $OCF_2CHF_2$, $OCF_2CHFCl$, $OCF_2CHFBr$, $OCF_2CHFI$, $OCFClCHFCl$ or $SCF_2CHF_2$.

43. Composition of Claim 42 wherein $X_2$ and $X_3$ are H, Cl or $CH_3$ and R is $OCF_2CHF_2$, $OCF_2CHFCl$ or $OCF_2CHFBr$.

44. Composition of Claim 43 wherein $X_1$ and $X_2$ are both Cl, and $X_3$ is H.

45. Composition of Claim 43 wherein $X_2$ and $X_3$ are both Cl and $X_1$ is H.

46. Composition of Claim 43 wherein $X_1$ and $X_3$ are both $CH_3$, and $X_2$ is H.

47. Composition of Claim 43 wherein $X_2$ and $X_3$ are both $CH_3$, and $X_1$ is H.

48. Composition of Claim 43 wherein $X_1$, $X_2$ and $X_3$ are all $CH_3$.

**Patentansprüche**

1. Verbindung der Formel

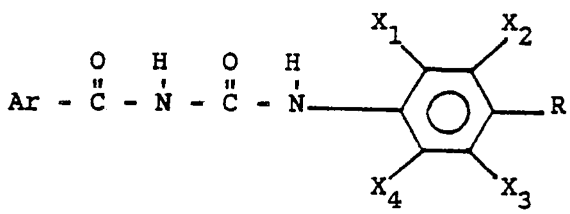

worin Ar ein substituiertes Phenylradikal ist, in dem die Substituenten Chlor oder Fluor sind, mit der Maßgabe, daß mindestens ein Substituent in ortho-Stellung zur Carbonylgruppe ist; $X_1$, $X_2$, $X_3$ und $X_4$ sind unabhängig voneinander H, Halogen, $C_1$—$C_3$-Alkyl, $C_1$—$C_3$-Alkoxy, $C_1$—$C_3$-Alkylcarbonyl, $C_1$—$C_3$-Alkoxycarbonyl, $C_1$—$C_3$-Alkylthio, mit der Maßgabe, daß mindestens zwei der Substituenten $X_1$, $X_2$, $X_3$ oder $X_4$ von H verschieden sind, und (a) wenn $X_1$ und $X_3$ H sind, $X_2$ und $X_4$ nicht beide Halogen sind, und (b) wenn $X_2$ und $X_3$ H sind, $X_1$ und $X_4$ nicht beide Halogen sind; und R eine $C_1$—$C_4$-Halogenalkyl-, $C_1$—$C_4$-Halogenalkoxy- oder $C_1$—$C_4$-Halogenalkylthiogruppe ist.

2. Verbindung nach Anspruch 1 mit der Formel

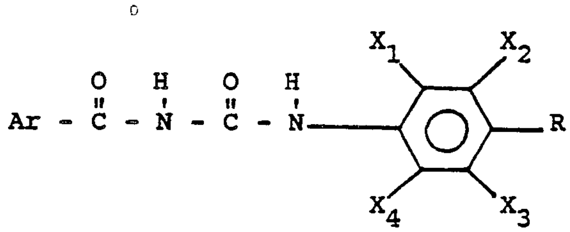

worin $X_1$ und $X_4$ Wasserstoff und $X_2$ und $X_3$ Halogen sind.

3. Verbindung nach Anspruch 2 mit der Formel

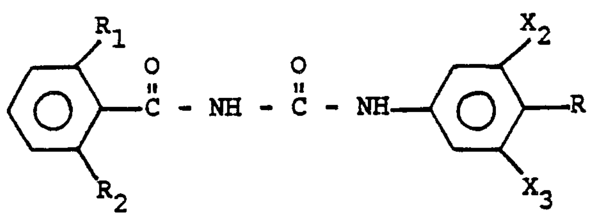

worin $R_1$ F oder Cl und $R_2$ H, F oder Cl ist.

4. Verbindung nach Anspruch 3, dadurch gekennzeichnet, daß $X_2$ und $X_3$ unabhängig voneinander F, Cl oder Br sind und R $OCF_3$, $OCF_2CHCl_2$, $OCF_2CHF_2$, $OCF_2CHFCl$, $OCF_2CHFBr$, $OCF_2CHFJ$, OCFClCHFCl oder $SCF_2CHF_2$ ist.

5. Verbindung nach Anspruch 4, dadurch gekennzeichnet, daß $R_1$ und $R_2$ beide F sind, $X_2$ und $X_3$ beide Cl sind, und R die Bedeutung $OCF_2CHF_2$ besitzt.

6. Verbindung nach Anspruch 4, dadurch gekennzeichnet, daß $R_1$ und $R_2$ beide F sind, $X_2$ und $X_3$ beide Chlor sind, und R die Bedeutung $OCF_2CHFCl$ besitzt.

7. Verbindung nach Anspruch 4, dadurch gekennzeichnet, daß $R_1$ und $R_2$ beide F sind, $X_2$ und $X_3$ beide Cl sind, und R die Bedeutung $OCF_2CHFBr$ besitzt.

8. Verbindung nach Anspruch 4, dadurch gekennzeichnet, daß $R_1$ Cl ist, $R_2$ H ist, $X_2$ und $X_3$ beide Cl sind, und R die Bedeutung $OCF_2CHF_2$, $OCF_2CHFCl$ oder $OCF_2CHFBr$ besitzt.

9. Verbindung nach Anspruch 4, dadurch gekennzeichnet, daß $R_1$ Cl ist, $R_2$ F ist, $X_2$ und $X_3$ beide Cl sind, und R die Bedeutung $OCF_2CHF_2$, $OCF_2CHFCl$ oder $OCF_2CHFBr$ besitzt.

10. Verbindung nach Anspruch 1 mit der Formel

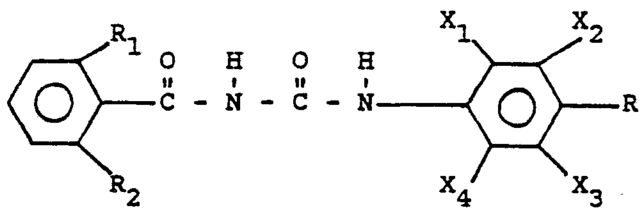

worin $R_1$ F oder Cl ist, $R_2$ H, F oder Cl ist, $X_1$, $X_2$, $X_3$ und $X_4$ unabhängig voneinander H, Halogen, Alkyl oder Alkoxy sind und R eine $C_1$—$C_4$-Halogenalkyl-, Halogenalkoxy- oder Halogenalkylthiogruppe ist.

11. Verbindung nach Anspruch 10, dadurch gekennzeichnet, daß $X_1$, $X_2$, $X_3$ und $X_4$ unabhängig voneinander H, Cl, F, Br, $CH_3$ oder $OCH_3$ sind und R $CF_3$, $OCF_3$, $OCF_2CHCl_2$, $OCF_2CHF_2$, $OCF_2CHFCl$, $OCF_2CHFBr$, $OCF_2CHFJ$, OCFClCHFCl oder $SCF_2CHF_2$ ist.

12. Verbindung nach Anspruch 11 mit der Formel

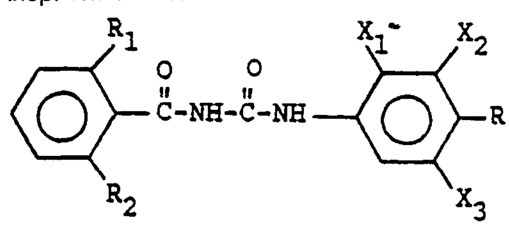

worin $R_1$ Fluor oder Chlor ist, $R_2$ H, F oder Cl ist, $X_1$ H, Cl oder $CH_3$ ist, $X_2$ H, Cl, $CH_3$ oder $OCH_3$ ist, $X_3$ H, Cl, $CH_3$ oder $OCH_3$ ist und R die Bedeutung $OCF_2CHF_2$, $OCF_2CHFCl$ oder $OCF_2CHFBr$ besitzt.

13. Verbindung nach Anspruch 12, dadurch gekennzeichnet, daß $R_1$ und $R_2$ beide F sind, $X_1$ und $X_2$ beide Cl sind, $X_3$ H ist und R die Bedeutung $OCF_2CHF_2$, $OCF_2CHFCl$ oder $OCF_2CHFBr$ besitzt.

14. Verbindung nach Anspruch 12, dadurch gekennzeichnet, daß $R_1$ und $R_2$ beide F sind, $X_1$ H ist, $X_2$ und $X_3$ beide $CH_3$ sind und R die Bedeutung $OCF_2CHFCl$ besitzt.

15. Verbindung nach Anspruch 12, dadurch gekennzeichnet, daß $R_1$ und $R_2$ beide F sind, $X_1$ H ist, $X_2$ und $X_3$ beide $CH_3$ sind und R $OCF_2CHF_2$ ist.

16. Verbindung nach Anspruch 12, dadurch gekennzeichnet, daß $R_1$ und $R_2$ beide F sind, $X_1$ H ist, $X_2$ und $X_3$ beide $CH_3$ sind und R $OCF_2CHFBr$ ist.

17. Verbindung nach Anspruch 12, dadurch gekennzeichnet, daß $R_1$ Cl ist, $R_2$ F ist, $X_1$ H ist, $X_2$ und $X_3$ beide $CH_3$ sind und R $OCF_2CHF_2$, $OCF_2CHFCl$ oder $OCF_2CHFBr$ ist.

18. Verbindung nach Anspruch 12, dadurch gekennzeichnet, daß $R_1$ und $R_2$ beide F sind, $X_1$ und $X_3$ beide $CH_3$ sind, $X_2$ H ist und R die Bedeutung $OCF_2CHF_2$, $OCF_2CHFCl$ oder $OCF_2CHFBr$ besitzt.

19. Verbindung nach Anspruch 12, dadurch gekennzeichnet, daß $R_1$ und $R_2$ beide F sind, $X_1$, $X_2$ und $X_3$ $CH_3$ sind und R die Bedeutung $OCF_2CHF_2$, $OCF_2CHFCl$ oder $OCF_2CHFBr$ besitzt.

20. Verbindung nach Anspruch 12, dadurch gekennzeichnet, daß $X_1$ Methyl ist und $X_2$ und $X_3$ Chlor sind.

21. Verbindung der Formel

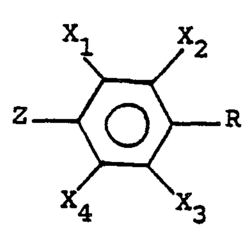

worin $X_1$, $X_2$, $X_3$ und $X_4$ unabhängig voneinander H, Halogen, $C_1$—$C_3$-Alkyl, $C_1$—$C_3$-Alkoxy, $C_1$—$C_3$-Alkylcarbonyl, $C_1$—$C_3$-Alkoxycarbonyl, $C_1$—$C_3$-Alkylthio sind, mit der Maßgabe, daß mindestens zwei der Substituenten $X_1$, $X_2$, $X_3$ oder $X_4$ von H verschieden sind und (a) wenn $X_1$ und $X_3$ H sind, $X_2$ und $X_4$ nicht beide Halogen sind, und (b) wenn $X_2$ und $X_3$ H sind, $X_1$ und $X_4$ nicht beide Halogen sind; R eine $C_1$—$C_4$-Halogenalkyl-, $C_1$—$C_4$-Halogenalkoxy- oder $C_1$—$C_4$-Halogenalkylthiogruppe ist und Z $NH_2$, —N=C=O,

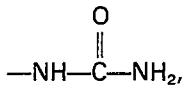, N=C=S oder 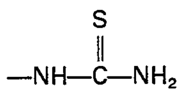 ist.

22. Verbindung nach Anspruch 21, dadurch gekennzeichnet, daß $X_1$ und $X_4$ Wasserstoff sind und $X_2$ und $X_3$ Halogen sind.

23. Verbindung nach Anspruch 22, dadurch gekennzeichnet, daß $X_2$ und $X_3$ unabhängig voneinander Fluor, Chlor oder Brom sind und R $OCF_3$, $OCF_2CHCl_2$, $OCF_2CHF_2$, $OCF_2CHFCl$, $OCF_2CHFBr$, $OCF_2CHFJ$, $OCFClCHFCl$ oder $SCF_2CHF_2$ ist.

24. Verbindung nach Anspruch 23, dadurch gekennzeichnet, daß $X_2$ und $X_3$ beide Chlor sind und R $OCF_2CHF_2$ ist.

25. Verbindung nach Anspruch 23, dadurch gekennzeichnet, daß $X_2$ und $X_3$ beide Cl sind und R die Bedeutung $OCF_2CHFCl$ besitzt.

26. Verbindung nach Anspruch 23, dadurch gekennzeichnet, daß $X_2$ und $X_3$ beide Cl sind und R die Bedeutung $OCF_2CHFBr$ besitzt.

27. Verbindung nach Anspruch 21, dadurch gekennzeichnet, daß $X_1$ und $X_2$ beide Cl sind, $X_3$ und $X_4$ H sind und R die Bedeutung $OCF_2CHF_2$, $OCF_2CHFCl$ oder $OCF_2CHFBr$ besitzt.

28. Verbindung nach Anspruch 21, dadurch gekennzeichnet, daß $X_1$ und $X_4$ beide H sind, $X_2$ und $X_3$ beide $CH_3$ sind und R die Bedeutung $OCF_2CHFCl$ besitzt.

29. Verbindung nach Anspruch 21, dadurch gekennzeichnet, daß $X_1$ und $X_4$ H sind, $X_2$ und $X_3$ beide $CH_3$ sind und R die Bedeutung $OCF_2CHF_2$ besitzt.

30. Verbindung nach Anspruch 21, dadurch gekennzeichnet, daß $X_1$ und $X_4$ H sind, $X_2$ und $X_3$ beide $CH_3$ sind und R $OCF_2CHFBr$ ist.

31. Verbindung nach Anspruch 21, dadurch gekennzeichnet, daß $X_1$ und $X_3$ beide $CH_3$ sind, $X_2$ und $X_4$ H sind und R die Bedeutung $OCF_2CHF_2$, $OCF_2CHFCl$ oder $OCF_2CHFBr$ besitzt.

32. Verbindung nach Anspruch 21, dadurch gekennzeichnet, daß $X_1$, $X_2$ und $X_3$ $CH_3$ sind, $X_4$ H ist und R die Bedeutung $OCF_2CHF_2$, $OCF_2CHFCl$ oder $OCF_2CHFBr$ besitzt.

33. Verfahren zur Abtötung und/oder Bekämpfung von Insekten, dadurch gekennzeichnet, daß man auf die Insekten, Insektenlarven oder ihre Habitate eine insektizid wirksame Menge einer Verbindung appliziert mit der Formel

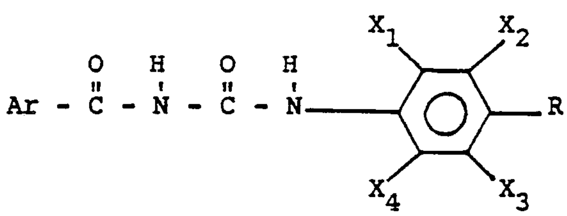

worin Ar ein substituiertes Phenylradikal ist, worin die Substituenten Chlor oder Fluor sind, mit der Maßgabe, daß mindestens ein Substituent in ortho-Stellung zur Carbonylgruppe steht; $X_1$, $X_2$, $X_3$ und $X_4$ unabhängig voneinander H, Halogen, $C_1$—$C_3$-Alkyl, $C_1$—$C_3$-Alkoxy, $C_1$—$C_3$-Alkylcarbonyl, $C_1$—$C_3$-Alkoxycarbonyl, $C_1$—$C_3$-Alkylthio sind, mit der Maßgabe, daß mindestens zwei der Substituenten $X_1$, $X_2$, $X_3$ oder $X_4$ von H verschieden sind und (a) wenn $X_1$ und $X_3$ H sind, $X_2$ und $X_4$ nicht beide Halogen sind, und (b) wenn $X_2$ und $X_3$ H sind, $X_1$ und $X_4$ nicht beide Halogen sind; und R eine $C_1$—$C_4$-Halogenalkyl-, $C_1$—$C_4$-Halogenalkoxy- oder $C_1$—$C_4$-Halogenalkylthiogruppe ist.

34. Verfahren nach Anspruch 33, dadurch gekennzeichnet, daß die verwendete Verbindung die Formel

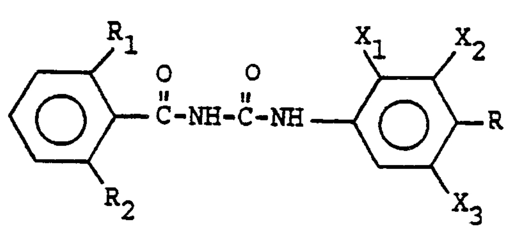

besitzt, worin $R_1$ F oder Cl ist; $R_2$ H, F oder Cl ist; $X_1$ H, Cl oder $CH_3$ ist; $X_2$ und $X_3$ H, Cl, $CH_3$ oder $OCH_3$ sind

und R die Bedeutung $OCF_3$, $OCF_2CHF_2$, $OCF_2CHFCl$, $OCF_2CHFBr$, $OCF_2CHFJ$, $OCFClCHFCl$ oder $SCF_2CHF_2$ besitzt.

35. Verfahren nach Anspruch 34, dadurch gekennzeichnet, daß $X_2$ und $X_3$ H, Cl oder $CH_3$ sind und R die Bedeutung $OCF_2CHF_2$, $OCF_2CHFCl$ oder $OCF_2CHFBr$ besitzt.

36. Verfahren nach Anspruch 35, dadurch gekennzeichnet, daß $X_1$ und $X_2$ beide Cl sind und $X_3$ H ist.

37. Verfahren nach Anspruch 35, dadurch gekennzeichnet, daß $X_2$ und $X_3$ beide Cl sind und $X_1$ H ist.

38. Verfahren nach Anspruch 35, dadurch gekennzeichnet, daß $X_1$ und $X_3$ beide $CH_3$ sind und $X_2$ H ist.

39. Verfahren nach Anspruch 35, dadurch gekennzeichnet, daß $X_2$ und $X_3$ beide $CH_3$ sind, und $X_1$ H ist.

40. Verfahren nach Anspruch 35, dadurch gekennzeichnet, daß $X_1$, $X_2$ und $X_3$ alle $CH_3$ bedeuten.

41. Zusammensetzung enthaltend eine insektizid wirksame Menge von mindestens einer Verbindung mit der Formel

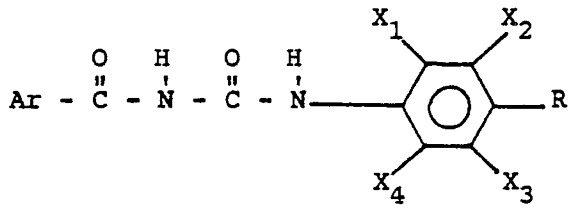

worin Ar ein substituiertes Phenylradikal ist, worin die Substituenten Chlor oder Fluor sind, mit der Maßgabe, daß mindestens ein Substituent in ortho-Stellung zur Carbonylgruppe steht; $X_1$, $X_2$, $X_3$ und $X_4$ unabhängig voneinander H, Halogen, $C_1$—$C_3$-Alkyl, $C_1$—$C_3$-Alkoxy, $C_1$—$C_3$-Alkylcarbonyl, $C_1$—$C_3$-Alkoxycarbonyl, $C_1$—$C_3$-Alkylthio sind, mit der Maßgabe, daß mindestens zwei der Substituenten $X_1$, $X_2$, $X_3$ oder $X_4$ von H verschieden sind und (a) wenn $X_1$ und $X_3$ H sind, $X_2$ und $X_4$ nicht beide Halogen sind und (b) wenn $X_2$ und $X_3$ H sind, $X_1$ und $X_4$ nicht beide Halogen sind; und R eine $C_1$—$C_4$-Halogenalkyl-, $C_1$—$C_4$-Halogenalkoxy- oder $C_1$—$C_4$-Halogenalkylthiogruppe ist.

42. Zusammensetzung nach Anspruch 41, dadurch gekennzeichnet, daß die Verbindung die Formel

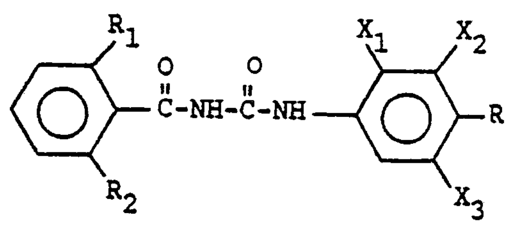

besitzt, worin $R_1$ F oder Cl ist; $R_2$ H, F oder Cl ist; $X_1$ H, Cl oder $CH_3$ ist; $X_2$ und $X_3$ H, Cl, $CH_3$ oder $OCH_3$ sind und R $OCF_3$, $OCF_2CHF_2$, $OCF_2CHFCl$, $OCF_2CHFBr$, $OCF_2CHFJ$, $OCFClCHFCl$ oder $SCF_2CHF_2$ ist.

43. Zusammensetzung nach Anspruch 42, dadurch gekennzeichnet, daß $X_2$ und $X_3$ H, Cl oder $CH_3$ sind und R die Bedeutung $OCF_2CHF_2$, $OCF_2CHFCl$ oder $OCF_2CHFBr$ besitzt.

44. Zusammensetzung nach Anspruch 43, dadurch gekennzeichnet, daß $X_1$ und $X_2$ beide Cl sind und $X_3$ H ist.

45. Zusammensetzung nach Anspruch 44, dadurch gekennzeichnet, daß $X_2$ und $X_3$ beide Cl sind und $X_1$ H ist.

46. Zusammensetzung nach Anspruch 44, dadurch gekennzeichnet, daß $X_1$ und $X_3$ beide $CH_3$ sind und $X_2$ H ist.

47. Zusammensetzung nach Anspruch 44, dadurch gekennzeichnet, daß $X_2$ und $X_3$ beide $CH_3$ sind und $X_1$ H ist.

48. Zusammensetzung nach Anspruch 44, dadurch gekennzeichnet, daß $X_1$, $X_2$ und $X_3$ alle $CH_3$ sind.

**Revendications**

1. Composé répondant à la formule

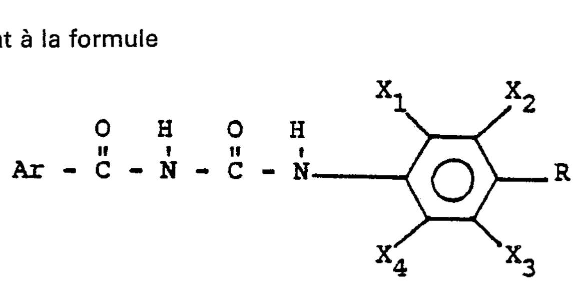

dans laquelle Ar est un radical phényle substitué dans lequel les substituants sont le chlore ou le fluor, à condition qu'au moins un substituant soit placé en position ortho par rapport au groupe carbonyle; $X_1$, $X_2$,

$X_3$ et $X_4$ sont individuellement H, un halogène, un groupe alkyle en $C_1$—$C_3$, alcoxy en $C_1$—$C_3$, (alkyl en $C_1$—$C_3$)-carbonyle, (alcoxy en $C_1$—$C_3$)-carbonyle, alkylthio en $C_1$—$C_3$, à condition qu'au moins deux des $X_1$, $X_2$, $X_3$ ou $X_4$ soient différents de H et que (a) lorsque $X_1$ et $X_3$ sont H, $X_2$ et $X_4$ ne puissent pas être tous les deux un halogène et (b) lorsque $X_2$ et $X_3$ sont H, $X_1$ et $X_4$ ne puissent pas être tous les deux un halogène; et R est un groupe alkyle halogéné en $C_1$—$C_4$, alcoxy halogéné en $C_1$—$C_4$ ou alkylthio halogéné en $C_1$—$C_4$.

2. Composé selon la revendication 1, répondant à la formule

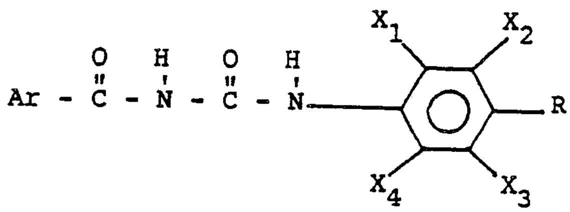

dans laquelle $X_1$ et $X_4$ sont l'hydrogène et $X_2$ et $X_3$ sont un halogène.

3. Composé selon la revendication 2, répondant à la formule

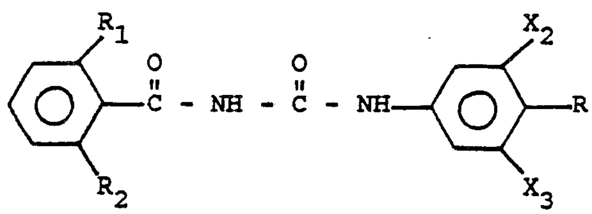

dans laquelle $R_1$ est F ou Cl et $R_2$ est H, F ou Cl.

4. Composé selon la revendication 3, dans lequel $X_2$ et $X_3$ sont indépendamment F, Cl ou Br et R est $OCF_3$, $OCF_2CHCl_2$, $OCF_2CHF_2$, $OCF_2CHFCl$, $OCF_2CHFBr$, $OCF_2CHFI$, OCFClCHFCl ou $SCF_2CHF_2$.

5. Composé selon la revendication 4, dans lequel $R_1$ et $R_2$ sont tous les deux F, $X_2$ et $X_3$ sont tous les deux Cl et R est $OCF_2CHF_2$.

6. Composé selon la revendication 4, dans lequel $R_1$ et $R_2$ sont tous les deux F, $X_2$ et $X_3$ sont tous les deux Cl et R est $OCF_2CHFCl$.

7. Composé selon la revendication 4, dans lequel $R_1$ et $R_2$ sont tous les deux F, $X_2$ et $X_3$ sont tous les deux Cl, et R est $OCF_2CHFBr$.

8. Composé selon la revendication 4, dans lequel $R_1$ est Cl, $R_2$ est H, $X_2$ et $X_3$ sont tous les deux Cl et R est $OCF_2CHF_2$, $OCF_2CHFCl$ ou $OCF_2CHFBr$.

9. Composé selon la revendication 4, dans lequel $R_1$ est Cl, $R_2$ est F, $X_2$ et $X_3$ sont tous les deux Cl et R est $OCF_2CHF_2$, $OCF_2CHFCl$ ou $OCF_2CHFBr$.

10. Composé selon la revendication 1, répondant à la formule

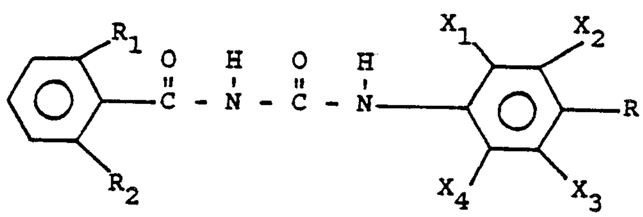

dans laquelle $R_1$ est F ou Cl, $R_2$ est H, F ou Cl, $X_1$, $X_2$, $X_3$ et $X_4$ sont individuellement H, un halogène, un groupe alkyle ou alcoxy et R est un groupe alkyle halogéné en $C_1$—$C_4$, alcoxy halogéné en $C_1$—$C_4$ ou alkylthio halogéné en $C_1$—$C_4$.

11. Composé selon la revendication 10, dans lequel $X_1$, $X_2$, $X_3$ et $X_4$ sont individuellement H, Cl, F, Br, $CH_3$ ou $OCH_3$ et R est $CF_3$, $OCF_3$, $OCF_2CHCl_2$, $OCF_2CHF_2$, $OCF_2CHFCl$, $OCF_2CHFBr$, $OCF_2CHFI$, OCFClCHFCl ou $SCF_2CHF_2$.

12. Composé selon la revendication 11, répondant à la formule

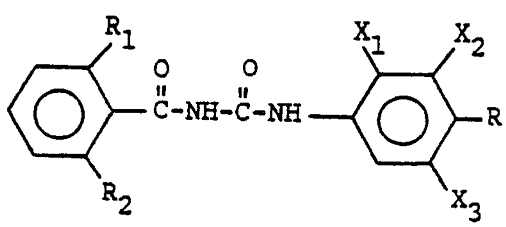

dans laquelle $R_1$ est F ou Cl, $R_2$ est H, F ou Cl, $X_1$ est H, Cl ou $CH_3$, $X_2$ est H, Cl, $CH_3$ ou $OCH_3$, $X_3$ est H, Cl, $CH_3$ ou $OCH_3$ et R est $OCF_2CHF_2$, $OCF_2CHFCl$ ou $OCF_2CHFBr$.

13. Composé selon la revendication 12, dans lequel $R_1$ et $R_2$ sont tous les deux F, $X_1$ et $X_2$ sont tous les deux Cl, $X_3$ est H et R est $OCF_2CHF_2$, $OCF_2CHFCl$ ou $OCF_2CHFBr$.

14. Composé selon la revendication 12, dans lequel $R_1$ et $R_2$ sont tous les deux F, $X_1$ est H, $X_2$ et $X_3$ sont tous les deux $CH_3$ et R est $OCF_2CHFCl$.

15. Composé selon la revendication 12, dans lequel $R_1$ et $R_2$ sont tous les deux F, $X_1$ est H, $X_2$ et $X_3$ sont tous les deux $CH_3$ et R est $OCF_2CHF_2$.

16. Composé selon la revendication 12, dans lequel $R_1$ et $R_2$ sont tous les deux R, $X_1$ est H, $X_2$ et $X_3$ sont tous les deux $CH_3$ et R est $OCF_2CHFBr$.

17. Composé selon la revendication 12, dans lequel $R_1$ est Cl, $R_2$ est F, $X_1$ est H, $X_2$ et $X_3$ sont tous les deux $CH_3$ et R est $OCF_2CHF_2$, $OCF_2CHFCl$ ou $OCF_2CHFBr$.

18. Composé selon la revendication 12, dans lequel $R_1$ et $R_2$ sont tous les deux F, $X_1$ et $X_3$ sont tous les deux $CH_3$, $X_2$ est H et R est $OCF_2CHF_2$, $OCF_2CHFCl$ ou $OCF_2CHFBr$.

19. Composé selon la revendication 12, dans lequel $R_1$ et $R_2$ sont tous les deux F, $X_1$, $X_2$ et $X_3$ sont $CH_3$ et R est $OCF_2CHF_2$, $OCF_2CHFCl$ ou $OCF_2CHFBr$.

20. Composé selon la revendication 12, dans lequel $X_1$ est le méthyle et $X_2$ et $X_3$ sont le chlore.

21. Composé répondant à la formule

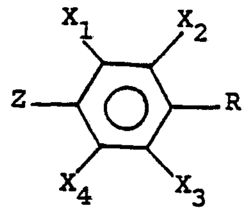

dans laquelle $X_1$, $X_2$, $X_3$ et $X_4$ sont indépendamment H, un halogène, un groupe alkyle en $C_1—C_3$, alcoxy en $C_1—C_3$, (alkyl en $C_1—C_3$)-carbonyle, (alcoxy en $C_1—C_3$)-carbonyle, alkylthio en $C_1—C_3$, à condition qu'au moins deux des $X_1$, $X_2$, $X_3$ ou $X_4$ soient différents de H et que (a) lorsque $X_1$ et $X_3$ sont H, $X_2$ et $X_4$ ne puissent pas être tous les deux un halogène et (b) lorsque $X_2$ et $X_3$ sont H, $X_1$ et $X_4$ ne puissent pas être tous les deux un halogène; R est un groupe alkyle halogéné en $C_{1-4}$, alcoxy halogéné en $C_{1-4}$ ou alkylthio halogéné en $C_{1-4}$ et Z est $NH_2$, $—N=C=O$,

$$—NH—\overset{\overset{O}{\|}}{C}—NH_2,\ N=C=S \text{ ou } —NH—\overset{\overset{S}{\|}}{C}—NH_2.$$

22. Composé selon la revendication 21, dans lequel $X_1$ et $X_4$ sont l'hydrogène et $X_2$ et $X_3$ sont un halogène.

23. Composé selon la revendication 22, dans lequel $X_2$ et $X_3$ sont indépendamment F, Cl ou Br et R est $OCF_3$, $OCF_2CHCl_2$, $OCF_2CHF_2$, $OCF_2CHFCl$, $OCF_2CHFBr$, $OCF_2CHFI$, $OCFClCHFCl$ ou $SCF_2CHF_2$.

24. Composé selon la revendication 23, dans lequel $X_2$ et $X_3$ sont tous les deux Cl et R est $OCF_2CHF_2$.

25. Composé selon la revendication 23, dans lequel $X_2$ et $X_3$ sont tous les deux Cl et R est $OCF_2CHFCl$.

26. Composé selon la revendication 23, dans lequel $X_2$ et $X_3$ sont tous les deux Cl et R est $OCF_2CHFBr$.

27. Composé selon la revendication 21, dans lequel $X_1$ et $X_2$ sont tous les deux Cl, $X_3$ et $X_4$ sont H et R est $OCF_2CHF_2$, $OCF_2CHFCl$ ou $OCF_2CHFBr$.

28. Composé selon la revendication 21, dans lequel $X_1$ et $X_4$ sont tous les deux H, $X_2$ et $X_3$ sont tous les deux $CH_3$ et R est $OCF_2CHFCl$.

29. Composé selon la revendication 21, dans lequel $X_1$ et $X_4$ sont H, $X_2$ et $X_3$ sont tous les deux $CH_3$ et R est $OCF_2CHF_2$.

30. Composé selon la revendication 21, dans lequel $X_1$ et $X_4$ sont H, $X_2$ et $X_3$ sont tous les deux $CH_3$ et R est $OCF_2CHFBr$.

31. Composé selon la revendication 21, dans lequel $X_1$ et $X_3$ sont tous les deux $CH_3$, $X_2$ et $X_4$ sont H et R est $OCF_2CHF_2$, $OCF_2CHFCl$ ou $OCF_2CHFBr$.

32. Composé selon la revendication 21, dans lequel $X_1$, $X_2$ et $X_3$ sont $CH_3$, $X_4$ est H et R est $OCF_2CHF_2$, $OCF_2CHFCl$ ou $OCF_2CHFBr$.

33. Procédé pour détruire et/ou controler les insectes, qui consiste à appliquer aux insectes, aux larves d'insectes ou à leurs biotopes, une quantité efficace du point de vue insecticide d'un composé répondant à la formule

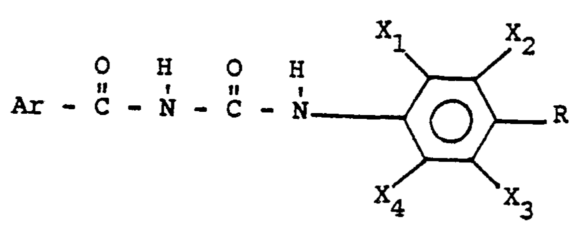

dans laquelle Ar est un radical phényle substitué dans lequel les substituants sont le chlore ou le fluor, à condition qu'au moins un substituant soit placé en position ortho par rapport au groupe carbonyle; $X_1$, $X_2$, $X_3$ et $X_4$ sont individuellement H, un halogène, un groupe alkyle en $C_1$—$C_3$, alcoxy en $C_1$—$C_3$, (alkyl en $C_1$—$C_3$) carbonyle, (alcoxy en $C_1$—$C_3$) carbonyle, alkylthio en $C_1$—$C_3$, à condition qu'au moins deux des $X_1$, $X_2$, $X_3$ ou $X_4$ soient différents de H, et que (a) lorsque $X_1$ et $X_3$ sont H, $X_2$ et $X_4$ ne puissent pas être tous les deux un halogène et (b) lorsque $X_2$ et $X_3$ sont H, $X_1$ et $X_4$ ne puissent pas être tous les deux un halogène; et R est un groupe alkyle halogéné en $C_{1-4}$, alcoxy halogéné en $C_{1-4}$ ou alkylthio halogéné en $C_{1-4}$.

34. Procédé selon la revendication 33, dans lequel le composé utilisé possède la formule

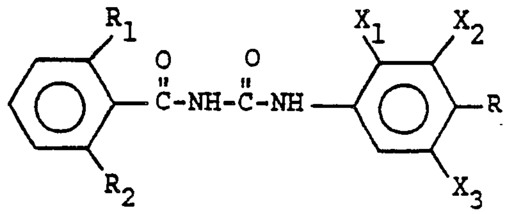

dans laquelle $R_1$ est F ou Cl; $R_2$ est H, F ou Cl; $X_1$ est H, Cl ou $CH_3$; $X_2$ et $X_3$ sont H, Cl, $CH_3$ ou $OCH_3$ et R est $OCF_3$, $OCF_2CHF_2$, $OCF_2CHFCl$, $OCF_2CHFBr$, $OCF_2CHFI$, $OCFClCHFCl$ ou $SCF_2CHF_2$.

35. Procédé selon la revendication 34, dans lequel $X_2$ et $X_3$ sont H, Cl ou $CH_3$ et R est $OCF_2CHF_2$, $OCF_2CHFCl$ ou $OCF_2CHFBr$.

36. Procédé selon la revendication 35, dans lequel $X_1$ et $X_2$ sont tous les deux Cl et $X_3$ est H.

37. Procédé selon la revendication 35, dans lequel $X_2$ et $X_3$ sont tous les deux Cl et $X_1$ est H.

38. Procédé selon la revendication 35, dans lequel $X_1$ et $X_3$ sont tous les deux $CH_3$ et $X_2$ est H.

39. Procédé selon la revendication 35, dans lequel $X_2$ et $X_3$ sont tous les deux $CH_3$ et $X_1$ est H.

40. Procédé selon la revendication 35, dans lequel $X_1$, $X_2$ et $X_3$ sont tous $CH_3$.

41. Composition qui comprend une quantité efficace du point de vue insecticide d'au moins un composé possédant la formule

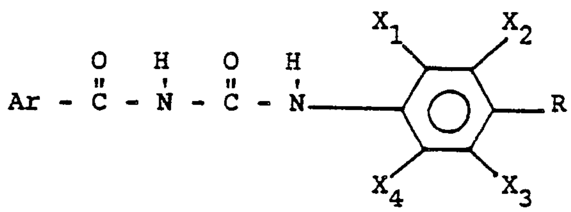

dans laquelle Ar est un radical phényle substitué dans lequel les substituants sont le chlore ou le fluor, à condition qu'au moins un substituant soit placé en position ortho par rapport au groupe carbonyle; $X_1$, $X_2$, $X_3$ et $X_4$ sont individuellement H, un halogène, un groupe alkyle en $C_1$—$C_3$, alcoxy en $C_1$—$C_3$, (alkyl en $C_1$—$C_3$)-carbonyle, (alcoxy en $C_1$—$C_3$)-carbonyle, alkylthio en $C_1$—$C_3$, à condition qu'au moins deux des $X_1$, $X_2$, $X_3$ ou $X_4$ soient différents de H et que (a) lorsque $X_1$ et $X_3$ sont H; $X_2$ et $X_4$ ne puissent pas être tous les deux un halogène et (b) lorsque $X_2$ et $X_3$ sont H, $X_1$ et $X_4$ ne puissent pas être tous les deux un halogène; et R est un groupe alkyle halogéné en $C_{1-4}$, alcoxy halogéné en $C_{1-4}$ ou alkylthio halogéné en $C_{1-4}$.

42. Composition selon la revendication 41, dans laquelle le composé possède la formule

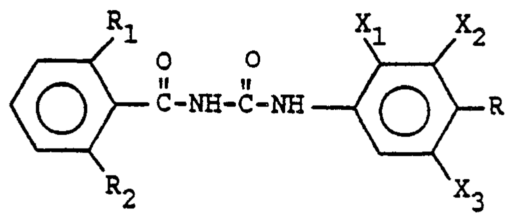

dans laquelle $R_1$ est F ou Cl; $R_2$ est H, F ou Cl; $X_1$ est H, Cl ou $CH_3$; $X_2$ et $X_3$ sont H, Cl, $CH_3$ ou $OCH_3$ et R est $OCF_3$, $OCF_2CHF_2$, $OCF_2CHFCl$, $OCF_2CHFBr$, $OCF_2CHFI$, $OCFClCHFCl$ ou $SCF_2CHF_2$.

43. Composition selon la revendication 42, dans laquelle $X_2$ et $X_3$ sont H, Cl ou $CH_3$ et R est $OCF_2CHF_2$, $OCF_2CHFCl$, ou $OCF_2CHFBr$.

44. Composition selon la revendication 43, dans laquelle $X_1$ et $X_2$ sont tous les deux Cl et $X_3$ est H.

45. Composition selon la revendication 43, dans laquelle $X_2$ et $X_3$ sont tous les deux Cl et $X_1$ est H.

46. Composition selon la revendication 43, dans laquelle $X_1$ et $X_3$ sont tous les deux $CH_3$ et $X_2$ est H.

47. Composition selon la revendication 43, dans laquelle $X_2$ et $X_3$ sont tous les deux $CH_3$ et $X_1$ est H.

48. Composition selon la revendication 43, dans laquelle $X_1$, $X_2$ et $X_3$ sont tous $CH_3$.